(19) **Europäisches Patentamt**

**European Patent Office**

**Office européen des brevets**

(11) **EP 0 635 030 B1**

(12) **EUROPEAN PATENT SPECIFICATION**

(45) Date of publication and mention
of the grant of the patent:
**21.07.2004 Bulletin 2004/30**

(51) Int Cl.[7]: **C07K 14/415**, A61K 39/395,
A61K 47/48

(21) Application number: **93912147.1**

(22) Date of filing: **08.04.1993**

(86) International application number:
**PCT/US1993/003292**

(87) International publication number:
**WO 1993/021232 (28.10.1993 Gazette 1993/26)**

(54) **IMMUNOTOXINS DIRECTED AGAINST c-erbB-2 (HER-2/neu) RELATED SURFACE ANTIGENS**

GEGEN C-ERB B-2 (HER-2/NEU) VEWANDTE OBERFLÄCHENANTIGENE GERICHTETE
IMMUNTOXINE

IMMUNOTOXINES DIRIGEES CONTRE DES ANTIGENES DE SURFACE APPARENTEES A
c-erbB-2 (HER-2/neu)

(84) Designated Contracting States:
**AT BE CH DE DK ES FR GB GR IE IT LI LU MC NL
PT SE**

(30) Priority: **10.04.1992 US 867728**

(43) Date of publication of application:
**25.01.1995 Bulletin 1995/04**

(73) Proprietor: **RESEARCH DEVELOPMENT
FOUNDATION
Carson City, Nevada 89703 (US)**

(72) Inventors:
• **ROSENBLUM, Michael G.
Houston, TX 77071 (US)**
• **SHAWVER, Laura K.
San Fransisco, CA 94112-1933 (US)**

(74) Representative: **Wilkinson, Stephen John et al
Stevens, Hewlett & Perkins
1 St. Augustine's Place
Bristol BS1 4UD (GB)**

(56) References cited:
**WO-A-93/03741          US-A- 5 091 513**

• **MAIER, LISA A. ET AL: "Requirements for the
internalization of a murine monoclonal antibody
directed against the HER-2/neu gene product
c-erbB-2" CANCER RES. (1991), 51(19), 5361-9
CODEN: CNREA8;ISSN: 0008-5472, 1991, pages
5361-5369, XP002063210**

• **Anticancer Research, Volume 10, Number 5A,
issued September-October 1990, R. TECCE et
al., "Production and Characterization of
Immunotoxins to Distinct Epitopes of the
Extracellular Domain of the HER-2 gp185", page
1454, see entire Abstract.**
• **Oncogene, Volume 4, issued 1989, S.J.
McKENZIE et al., "Generation and
Characterization of Monoclonal Antibodies
Specific for the Human Neu Oncogene Product,
p185", pages 543-548, see entire document.**
• **Science, Volume 238, issued 20 November 1987,
E.S. VITETTA et al., "Redesigning Nature's
Poisons to Create Anti-tumor Reagents", pages
1098-1104, see entire document.**
• **Blood, Volume 66, Number 3, issued September
1985, A.L. HOWELL et al., "Monoclonal
Antibody-Mediated Cytotoxicity of Human
Myeloid Leukemia Cells: in In Vitro Model for
Estimating Efficiency and Optimal Conditions
for Cytolysis", pages 649-654, see entire
document.**
• **Blood, Volume 75, Number 5, issued 01 March
1990, E.D. BALL et al., "Autologous Bone
Marrow Transplantation for Acute Myeloid
Leukemia Using Monoclonal Antibody-Purged
Bone Marrow", pages 1199-1206, see entire
document.**

- **Cancer Research, Volume 51, issued 15 May 1991, LANGTON et al., "An Antigen Immunologically Related to the External Domain of gp185 is Shed from Nude Mouse Tumors Overexpressing the c-erb-2(HER-2/neu) Oncogene", pages 2593-2598, see entire document.**
- **Cancer Research, Volume 52, issued 01 April 1992, DeSANTES et al., "Radiolabeled Antibody Targeting of the HER-2/neu Oncoprotein", pages 1916-1923, see entire document.**
- **Annals of Internal Medicine, Volume 111, Number 7, issued 01 October 1989, R.O. DILLMAN, "Monoclonal Antibodies for Treating Cancer", pages 592-603, see entire document.**

Remarks:

The file contains technical information submitted after the application was filed and not included in this specification

**Description**

BACKGROUND OF THE INVENTION

Field of the Invention

The present invention relates generally to the field of treatment of neoplastic disease. More specifically, the present invention relates to novel immunoconjugates and their use in the treatment of neoplastic disease.

Description of the Related Art

Neoplastic disease is one of the leading causes of mortality and morbidity in the Western World. Neoplastic conditions, e.g., diseases or "cancers", share at least one characteristic, i.e., the involvement of defects in the cellular growth regulatory process.

The process by which normal cells are transformed into malignant cells has been a subject of intense study for decades. More recently, study has focused on the role of oncogenes in the cancer process. Oncogenes are genes that have the ability to transform eukaryotic cells so that they grow in a manner analogous to tumor cells.

An oncogene is created when a normal gene or proto-oncogene is mutated, rearranged, or amplified. One such oncogene is the c-erbB-2(HER-2/neu) proto-oncogene. Hereinafter this oncogene will be referred to as c-erbB-2. This gene encodes a protein similar to the epidermal growth factor receptor. Amplification of this proto-oncogene can result in a cascade of cellular events leading to unregulated cell growth.

Antibodies are proteins produced by the immune system of an animal, normally in response to foreign antigens or antigenic determinants. Antibodies bind to the specific antigen to which they are directed. The development of specific monoclonal antibodies has provided investigators with a possible means of selectively targeting therapeutic agents to cells which overexpress defined antigens.

Tecce et al (Anti-Cancer Research 1990, Vol. 10(5A) page 1454) have produced immunotoxins to HER-2 product which employ two monoclonal antibodies recognizing different epitopes on gp185 that have been conjugated to the plant toxin saporin. Sivam et al (Cancer Research 47, 3169-3175, 15 June 1987) have covalently linked gelonin to monoclonal antibody 9.2.27, directed to a human melanoma-associated glycoprotein/proteoglycan.

Overexpression of the c-erbB-2 proto-oncogene in neoplastic transformation has been postulated. Several types of human cancers including some mammary carcinomas and some ovarian carcinomas have an amplified c-erbB-2 gene. Moreover, amplification and subsequent overexpression of the c-erbB-2 gene has been correlated with poor disease prognosis. Thus, there exists a great need and desire in this art for a method of selectively targeting a chemotherapeutic agent to a cell which exhibits overexpression of the c-erbB-2 oncogene to modulate growth of cells which overexpress the protein. The present invention provides means for accomplishing this.

SUMMARY OF THE INVENTION

The present invention provides a novel composition comprising a conjugate of a protein targeting moiety, e.g., an antigen binding region, exhibiting binding specificity for an antigen domain for c-erbB-2 protein and a plant derived toxin, wherein said toxin is selected from the group consisting of gelonin and full length recombinant gelonin. Such a composition can act as an immunotoxin to specifically target a cell growth modulator to tumor cells overexpressing the c-erbB-2 protein.

Thus, in one embodiment of the present invention, there is provided a new composition of matter comprising a conjugate of a protein targeting moiety with binding specificity for the c-erbB-2 protein, e.g., TAb 250 monoclonal antibody, a $V_H$ segment of an antibody, intact immunoglobulin heavy chain, a $V_L$ segment of an antibody, intact immunoglobulin light chain, intact immunoglobulin heavy chain or single chain antibody, and a toxic moiety. The toxic moiety may be a biological response modifier. In one particular embodiment, the toxic moiety is a plant derived toxin having a much lower cellular effect when not conjugated to the targeting moiety. This toxin can be selected from the group consisting of native toxin or recombinant toxin. In a further embodiment, the toxic moiety is gelonin.

Another embodiment of the present invention provides a pharmaceutical composition for treating a neoplastic condition, *e.g.*, disease, which is characterized by amplification or overexpression of the c-erbB-2 oncogene in cells, comprising administering a cytocidally effective dose of an immunotoxin of the present invention to an individual in need of such treatment.

Another embodiment provides a composition further comprising a pharmaceutically acceptable vehicle.

Another embodiment provides a method of killing tumor cells *in vitro,* typically followed by reintroduction into a host. For example, in treating a bone marrow neoplastic condition, bone marrow is removed from an individual having the neoplastic disease and treated with a composition of the present invention.

Another embodiment provides a composition for use in the preparation of a medicament for the treatment of overexpression of c-erbB-2 protein or neoplastic cells, e.g., mammary carcinoma cells, ovarian carcinoma cells, lung carcinoma cells, salivary gland carcinoma cells, gastric tumor cells, colon adenocarcinoma cells and bone marrow leukemia cells

Another embodiment provides an *in vitro* method of treating a neoplastic cell, the method comprising administering an effective dose of the composition to the cells.

In another embodiment of the present invention there is provided a method of preventing recurrence of neoplastic disease. The recurrence is prevented by administration of a cytocidally effective treatment of the targeted toxin, *e.g.*, an immunotoxin such as TAb 250 antibody-gelonin.

In still another embodiment of the present invention, there are provided new compositions of matter comprising fusion constructs of targeting moieties with binding-affinity for c-erbB-2 protein and a toxic moiety. Preferably, the targeting moiety is an antibody which recognizes an extracellular epitope of c-erbB-2, *e.g.*, TAb 250, and the toxic moiety is relatively inert when applied separately from the targeting moiety, *e.g.*, gelonin. In other embodiments of the present invention there are provided methods of extending the survival time of a tumor bearing mammal by administration of targeted toxins of the present invention to this mammal and also a method of retarding the rate of growth of tumors by administering targeted toxins of the present invention. Typically, the targeted toxins will be targeted by an immunological binding region, *e.g.*, an antibody binding segment. Additionally provided is a pharmaceutical composition comprising an immunotoxin consisting essentially of a toxic moiety conjugated to a monoclonal antibody. Most preferably, the antibody is TAb 250 and the toxic moiety is gelonin.

## BRIEF DESCRIPTION OF THE DRAWINGS

Figure 1 demonstrates the effects of ZME antibody, TAb 250 antibody or immunonconjugates of TAb 250 and gelonin on SKOV-3 cells as measured by ELISA.

Figure 2 demonstrates the cytotoxicity of the TAb 250 gelonin construct on SKOV-3 cells.

Figure 3 demonstrates the competition of relevant versus irrelevant antibody with the conjugate on SKOV-3 cells.

Figure 4 demonstrates the dose response relationship and the effects of the TAb 250-gelonin conjugate on SKOV-3 cells.

Figure 5 demonstrates the cytotoxicity of TAb 250 and the TAb 250-gelonin conjugate on SKOV-3 cells.

Figure 6 demonstrates the ability of the TAb 250 antibody to internalize in various cell lines.

Figure 7 demonstrates the cytotoxicity of the TAb 250-gelonin immunoconjugate in the MTT assay.

## DETAILED DESCRIPTION OF THE INVENTION

As used herein, a cellular targeting moiety is capable to selectively binding to a c-erbB-2 protein which is expressed on a cell, typically on its surface. This includes a protein exhibiting binding specifically for an antigen domain to the c-erbB-2 protein *e.g.*, intact antibodies or epitope binding fragments thereof. This encompasses both classical antibody molecules, chimeric versions, single chain, and modified antibody fragments which retain epitope binding specificity and affinity.

As used herein, the term "immunoglobulin" or "antibody peptide(s)" refers to an entire immunoglobulin or antibody or any functional binding fragment of an immunoglobulin molecule. Examples of such peptides include complete antibody molecules, antibody fragments, such as Fab, F(ab')$_2$, CDRs, $V_L$, $V_H$, and any other portion of an antibody, particularly those exhibiting antigen binding specificity or affinity. For instance, an IgG antibody molecule is composed of two light chains, each linked by disulfide bonds to two heavy chains. The two heavy chains are, in turn, linked to one another by disulfide bonds in an area known as the hinge region of the antibody. A single IgG molecule typically has a molecular weight of approximately 150-160 kD and contains two antigen binding sites. Fragments of these molecules, *e.g.*, heavy or light chains alone, can sometimes bind antigen. Antibodies, fragments of antibodies, and individual chains can be functionally equivalent to immunoglobulins.

A normal antibody heavy or light chain has an N-terminal (NH$_2$) variable (V) region, and a C-terminal (-COOH) constant (C) region. The heavy chain variable region is referred to as $V_H$ (including, for example, $V_\gamma$), and the light chain variable region is referred to as $V_L$ (including $V_\kappa$ or, $V_\lambda$). The variable region is the part of the molecule that binds to the antibody's cognate antigen, while the Fc region (the second and third domains of the C region) determines the antibody's effector function (*e.g.*, complement fixation, opsonization). Full-length immunoglobulin or antibody "light chains" (generally about 25 Kd, about 214 amino acids) are encoded by a variable region gene at the N-terminus (generally about 110 amino acids) and a κ (kappa) or λ (lambda) constant region gene at the COOH-terminus. Full-length immunoglobulin or antibody "heavy chains" (generally about 50 Kd, about 446 amino acids), are similarly encoded by a variable region gene (generally encoding about 116 amino acids) and one of the constant region genes, *e.g.* gamma (encoding about 330 amino acids). Typically, the "$V_L$" will include the portion of the light chain encoded by the $V_L$ and/or $J_L$ (J or joining region) gene segments, and the "$V_H$" will include the portion of the heavy chain encoded by the $V_H$, and/or $D_H$ (D or diversity region) and $J_H$ gene segments. *See generally,* Roitt, *et al., Immunology,* Chapter 6, (2d ed. 1989) and Paul; *Fundamental Immunology*, Raven Press (2d ed. 1989).

An immunoglobulin light or heavy chain variable region consists of a "framework" region interrupted by three hypervariable regions, also called complementarity-determining regions or CDRs. The extent of the framework region and CDRs have been defined (see, "Sequences of Proteins of Immunological Interest," E. Kabat, *et al.,* U.S. Department of Health and Human Services, (1987),

The sequences.of the framework regions of different light or heavy chains are relatively conserved within a species. The framework regions of an antibody, that is the combined framework regions of the constituent light and heavy chains, serve to position and align the CDRs in three dimensional space. The CDRs are primarily responsible for binding to an epitope of an antigen. The CDRs are typically referred to as CDR1, CDR2, and CDR3, numbered sequentially starting from the N-terminus.

The two types of light chains, $\kappa$ and $\lambda$, are referred to as isotypes. Isotypic determinants typically reside in the constant region of the light chain, also referred to as the $C_L$ in general, and $C_\kappa$ or $C_\lambda$ in particular. The constant region of the heavy chain molecule, also known as $C_H$, determines the isotype of the antibody. Antibodies are classified as IgM, IgD, IgG, IgA, and IgE depending on the heavy chain isotype. The isotypes are encoded in the mu ($\mu$), delta ($\Delta$), gamma ($\gamma$), alpha ($\alpha$), and epsilon ($\varepsilon$) segments of the heavy chain constant region, respectively. In addition, there are a number of $\gamma$ subtypes.

The heavy chain isotype determines different effector functions of the antibody, such as opsonization or complement fixation. In addition, the heavy chain isotype determines the secreted form of the antibody. Secreted IgG, IgD, and IgE isotypes are typically found in single unit or monomeric form. Secreted IgM isotype is found in pentameric form; secreted IgA can be found in both monomeric and dimeric form.

An $F(ab')_2$ fragment lacks the C-terminal portion of the heavy chain constant region, and usually has a molecular weight of approximately 110 kD. It retains two antigen binding sites and the interchain disulfide bonds in the hinge region, but it does not have the effector functions of an intact IgG molecule. An $F(ab')_2$ fragment may be obtained from an IgG molecule by proteolytic digestion with pepsin at pH 3.0-3.5 using standard methods such as those described in Harlow and Lane, *infra.*

An "Fab" fragment comprises a light chain and the N-terminal portion of the heavy chain which are linked together by disulfide bonds. It typically has a molecular weight of approximately 50 kD and contains a single antigen binding site. Fab fragments may be obtained from $F(ab')_2$ fragments by limited reduction, or from whole antibody by digestion with papain in the presence of reducing agents. (*See*, Harlow and Lane, *infra.*) In certain cases, the concentration of reducing agent necessary to maintain the activity of papain in the presence of atmospheric oxygen is sufficient to fully reduce the interchain disulfide bonds to the antibody. This can result in loss of antigen recognition. To circumvent this problem, papain may be activated and then exchanged into buffer containing a concentration of reducing agent compatible with maintaining antigen binding activity. The antibody digestion is typically carried out under an inert atmosphere to prevent deactivation of the papain.

The following protocol is an example of this process:

A) Activation of papain: Papain, supplied as 10 mg/ml $NH_4SO_4$ suspension, is dissolved in 10 mM EDTA, 20 mM cysteine, pH=8.0, to a final concentration of 2 mg/ml. The solution is degassed and allowed to incubate 2 hours at room temperature under nitrogen.
B) The activated papain is exchanged into 20 mM $NaPO_4$, pH=7.0, 150 mM NaCl, 10 mM EDTA, 30 $\mu$M DTT.
C) Digestion of antibody: 1 mg of activated papain is added for every 100 mg of antibody, and the solution is dialyzed against a large excess of 20 mM $NaPO_4$, pH=7.0, 150 mM NaCl, 10 mM EDTA, 30 $\mu$M DTT, with continuous helium sparging. Dialysis is used to maintain a molar excess of reducing agent during the course of the digestion.
D) After 2-4 hours at room temperature the digestion is terminated by addition of iodoacetamide.
E) Fab fragments are separated from undigested or partially digested antibody using standard chromatography methods.

As used herein, the terms "Fab", or any other antibody fragments, have similar classifications when applied to the present invention as to the general terms "antibodies" or "immunoglobulins". Thus, "mammalian" Fab protein, "chimeric Fab", and the like are used analogously to the corresponding definitions in general usage, and as set forth in the subsequent paragraphs.

As used herein, the term "chimeric antibodies" or "chimeric peptides" refer to those antibodies or antibody peptides wherein one portion of the peptide has an amino acid sequence that is derived from, or is homologous to, a corresponding sequence in an antibody or peptide derived from a first gene source, while the remaining segment of the chain(s) is homologous to corresponding sequences of another gene source. For example, a chimeric heavy chain antibody peptide may comprise a murine variable region and a human constant region. The two gene sources will typically be two separate species, but will occasionally involve one species.

Chimeric antibodies or peptides are typically produced using recombinant molecular and/or cellular techniques. In many cases, chimeric antibodies have variable regions of both light and heavy chains that mimic the variable regions of antibodies derived from one mammalian species, while the constant and/or framework portions are homologous to the sequences in antibodies derived from a second, different mammalian species.

As used herein, the definition of chimeric .antibody, however, is not limited to this example. A chimeric antibody is any antibody in which either or both of the heavy or light chains are composed of combinations of sequences mimicking the sequences in antibodies of different sources, whether these sources be differing classes, differing antigen responses, or differing species of origin, and whether or not the fusion point is at the variable/constant boundary. For example, chimeric antibodies can include antibodies where the framework and CDRs are from different sources. For example, non-human CDRs are integrated into human framework regions linked to a human constant region to make "humanized antibodies." *See, e.g.,* PCT Application Publication No. WO 87/02671; U.S. Patent No. 4,816,567; EP Patent Application 0173494; *Jones, et al., Nature*, 321:522-525 (1986) ; and Verhoeyen, *et al., Science,* 239:1534-1536 (1988).

As used herein, the term "human-like framework region" is a framework region for each antibody chain, and it usually comprises at least about 70 amino acid residues, typically 75 to 85 or more residues. The amino acid residues of the human-like framework region are at least about 80%, preferably about 80-85%, and most preferably more than 85% homologous with those in a human immunoglobulin. This shared feature with other endogenous antibodies is useful in generating a targeting moiety which introduces only a minor immune reaction, *e.g.*, a mechanism which minimizes response to "self" markers.

As used herein, the term "humanized" or "human-like immunoglobulin" refers to an immunoglobulin comprising a human-like framework region and a constant region that is substantially homologous to a human immunoglobulin constant region, *e.g.*, having at least about 80% or more, preferably about 85-90% or more and most preferably about 95% or more homology. Hence, most parts of a human-like immunoglobulin, except possibly the CDRs, are substantially homologous to corresponding parts of one or more native human immunoglobulin sequences.

As used herein, the term "hybrid antibody" refers to an antibody wherein each chain is separately homologous with reference to a mammalian antibody chain, but the combination represents a novel assembly so that two different antigens are recognized by the antibody. In hybrid antibodies, one heavy and light chain pair is homologous to that found in an antibody raised against one antigen recognition feature, e.g., epitope, while the other heavy and light chain pair is homologous to a pair found in an antibody raised against another epitope. This results in the property of multifunctional valency, i.e., ability to bind at least two different epitopes simultaneously. Such hybrids may, of course, also be formed using chimeric chains.

As used herein, the term "monoclonal antibody" means an antibody composition recognizing a discrete antigen determinant. It is not intended to be limited as regards the source of the antibody or the manner in which it is made.

Using standard methods that are well known in the art, the variable regions and CDRs may be derived from a hybridoma that produces a monoclonal antibody that is specific for c-erbB-2. The nucleic acid sequences of the present invention capable of ultimately expressing the desired chimeric antibodies can be formed from a variety of different nucleotide sequences (genomic or cDNA, RNA, synthetic oligonucleotides, etc.) and components (*e.g.,* V, J, D, and c regions), as well as by a variety of different techniques. Joining appropriate genomic sequences is presently a common method of production, but cDNA sequences may also be utilized (see, European Patent Publication No. 0239400 and Reichmann, *L., et* al., *Nature, 332*:323-327 (1988).

Human constant region DNA sequences are preferably isolated from immortalized B-cells, see *e.g.*, Heiter, *et al., Cell, 22*:197-207 (1980), incorporated by reference herein, but can be isolated or synthesized from a variety of other sources. The nucleotide sequence of a human immunoglobulin $C_{\gamma 1}$ gene is described in Ellison, *et al., Nucl. Acid. Res.*, *10*:4071 (1982); Beidler, *et al., J. Immunol.*, 141:4053 (1988); Liu, *et al., Proc. Natl. Acad. Sci. USA,* 84:3439 (1987).

The CDRs for producing the immunoglobulins of the present invention preferably are derived from monoclonal antibodies capable of binding to the desired antigen, c-erbB=2 protein, and produced in any convenient mammalian source, including, mice, rats, rabbits, hamsters, or other vertebrate host cells capable of producing antibodies by well known methods. Suitable source cells for the DNA sequences and host cells for immunoglobulin expression and secretion can be obtained from a number of sources, such as the American Type Culture Collection ("ATCC") ("Catalogue of Cell Lines and Hybridomas," Fifth edition (1985) Rockville, Maryland, U.S.A.

In addition to the chimeric antibody peptides specifically described herein, other "substantially homologous" modified immunoglobulins can be readily designed and manufactured utilizing various recombinant DNA techniques known to those skilled in the art. Modifications of the genes may be readily accomplished by a variety of well-known techniques, such as site-directed mutagenesis (see, Gillman and Smith, Gene, 8:81-97 (1979) and Roberts, *S., et al.,*

*Nature, 328*:731-734 (1987).

These modifications can include amino acid additions, deletions, substitutions, preferably conservative, and other changes in the sequence of the polypeptide while retaining the appropriate property or biological activity. Alternatively, polypeptide fragments comprising only a portion of the primary antibody structure and possessing binding and/or effector activities may be produced. Also because, like many genes, the immunoglobulin-related genes contain separate functional regions, each having one or more distinct biological activities, the genes may be fused to functional regions from other genes to produce fusion proteins (*e.g.*, immunotoxins) having novel properties or novel combinations of properties.

The cloned variable and constant regions can be isolated from plasmids and ligated together into a mammalian expression vector, *e.g.*, pSV2-neo, or pRSV-gpt, to form a functional transcription unit. These expression vectors can then be transfected into host cells. Mouse myeloma cells, such as SP 2/O or P3X cells, are a preferred host because they do not secrete endogenous immunoglobulin protein and contain all of the components used in immunoglobulin expression. Myeloma cells can be transfected using appropriate techniques as described above.

Other types of promoters and enhancers specific for other host cells are known in the art. *See*, Kameyoma, K., et *al., supra.* For example, the DNA sequence encoding the chimeric antibody amino acid sequence can be linked to yeast promoters and enhancers and transfected into yeast by methods well known in the art. *See,* Kriegler, *supra*.

This same approach can be taken to isolate the c-erbB-2 specific CDRs from one source such as one mammalian species and the framework regions of another source, such as a different mammalian species. The CDRs can then be ligated to the framework regions and constant regions to form a chimeric antibody. *See*, PCT No. GB88/00731 (1989), and U.S.S.N. 07/808,462, filed December 12, 1991,

The CDRs could be cloned in an expression vector comprising, for example, human framework and constant regions.

Another example is a recombinant DNA sequence comprising the heavy and/or light chain CDR1, CDR2, and CDR3 of one species, such as mouse, and the framework regions of human heavy chain to encode an antibody . specific for c-erbB-2. Other possibilities include using CDRs specific for c-erbB-2; using part of the variable region encompassing CDR1 and CDR2 from one mammalian species, and then ligating this sequence to another encoding the framework portions of a second mammalian species to the CDR3 of the first; or transfecting a host cell line with a recombinant DNA sequence encoding a c-erbB-2 specific heavy chain CDRs derived from a first mammalian species, interspersed within the framework of a second mammalian species with a light chain containing a variable region DNA sequence derived from.the first species and the constant region derived from the second species.

Recombinant DNA expression vectors comprising antibody sequences may be transfected by electroporation into host cells. Standard selection procedures are used to isolate clones that produce the c-erbB-2 specific chimeric antibody.

Antibodies may be expressed in an appropriate folded form, including single chain antibodies, from bacteria such as *E.* coli. *See*, Pluckthun, *Biotechnology,* 9:545 (1991); Huse, *et al., Science,* 246:1275 (1989); and Ward, et *al., Nature*, 341:544 (1989).

The antibody peptide sequences may be amplified for cloning by use of polymerase chain reaction, or PCR, a technique used to amplify a DNA sequence of interest using a thermostable DNA polymerase, such as Taq polymerase, and polymerase and oligonucleotide primers, all as described in PCR *Protocols,* ed. Innis, *et al.*, Academic Press, Inc. (1990). See also Orlandi, *supra* and Larrick, *et al.*, *Biotechnology,* 7:934 (1989).

The c-erbB-2 protein (also referred to here simply as c-erbB-2) is a 185 Kd (Kilodalton) membrane glycoprotein having tyrosine kinase activity and is related to, but distinct from, the epidermal growth factor receptor (EGFR). Like the EGFR protein, the c-erbB-2 protein has an extracellular domain that includes two cysteine-rich repeat clusters, a transmembrane domain, and an intracellular kinase domain. In addition, the amino acid sequence of the c-erbB-2 protein as well as the nucleotide sequence has been described by Coussens, *et al., Science,* 230:1132 (1985).

The c-erbB-2 protein is encoded by the c-erbB-2 oncogene described in 1985 by three different research groups: Semba, *et al., Proc. Natl. Acad. Sci USA,* 82:6497 (designating the gene as c-erbB-2); Coussens, *et al., supra,* (designating the gene as HER-2); and King, *et al., Science,* 229:1132 (designating the gene as v-erbB related). Thus, the c-erbB-2 gene sequence and its corresponding protein sequence are well-known and described in the art. The c-erbB-2 protein has a defined intracellular design, a transmembrane region, and an extracellular region. Typically, the targeting moieties of the present invention will bind to the extracellular region, which should be exposed to the exterior of the neoplastic cell. The targeting moiety will generally recognize a feature or features found there, including an antigen recognition sites, e.g., epitopes. The epitopes will often be directed to pure polypeptide epitopes, either linear peptide sequence determinants or conformational determinants, but can also be directed to epitopes having carbohydrate components. The epitopes can thus include combined protein/carbohydrate components, or carbohydrate components alone. Other modifications to the protein, normal or abnormal, will present important epitopic determinants, also.

Detection of the c-erbB-2 protein may be accomplished by well-known immunoassays employing antibodies specific to the d-erbB-2 protein, such as those described here. Such antibodies are commercially available, for example, from Chemicon International, Inc., Temecula, CA or may be prepared by standard immunological procedures. *See, e. g.*, Harlow and Lane, *Antibodies: A Laboratory Manual,* Cold Spring Harbor Publications, N. Y. (1988).

It is intended herein that the c-erbB-2 protein definition will also include those proteins developed from other host systems, *e.g.*, proteins that are immunologically related to the human c-erbB-2 protein. For example, a related rat gene (designated neu) has been reported in Schecter, *et al.*, *Science*, 229:976 (1985).

Useful epitopes to which antibodies may be easily raised include extracellular epitopes found on target cells. These epitopes will generally be protein epitopes, *e.g.,* linear or conformational epitopes of the protein as found on neoplastic cells. Other useful epitopes will include non-proteineous components including carbohydrate or other modifications, usually post-translational, found on the c-erbB-2 protein. Antibodies and other binding regions which exhibit binding specificity for overexpressed c-erbB-2 may be raised against fragments of the protein.

Mouse monoclonal antibodies have been made against the extracellular portion of c-erbB-2. One example of such an antibody is TAb 250, which is deposited with the American Type Culture Collection, Rockville, Maryland (ATCC) bearing Accession No. HB10646.

Alternatively, a protein targeting moiety may be derived from any other targeting method which exhibits affinity and specificity for a c-erbB expressing cell. For example, a ligand which is recognized and bound by the c-erbB-2 protein would be a useful targeting moiety. See, *e.g.,* Ciccodicola, *et al.* (1989) *Embo J.* 8:1987-1991; Ciardiello, *etal.* (1991) *Cancer Research* 51:1051-1054; and Ciardiello, *et al.* (1991) *P.N.A.S. USA* 88:7792-7796, which describe CRIP-TO, a molecule which appears to serve as a ligand for the EGF receptor, and will likely also bind with specificity to the c-erbB-2 protein.

In the case of the sequences described herein, it should be understood that variants of these sequences are also included, such a substitution, addition, and/or deletion mutations, or any other sequence possessing substantially similar binding activity to the sequences from which they are derived or otherwise similar to.

For this invention, an antibody or other peptide is specific for a c-erbB-2 protein if the antibody or peptide binds or is capable of binding c-erbB-2, *e.g.,* protein as measured or determined by standard antibody-antigen or ligand-receptor assays, for example, competitive assays, saturation assays, or standard immunoassays such as ELISA or RIA. This definition of specificity applies to single heavy and/or light chains, CDRs, fusion proteins or fragments of. heavy and/or light chains, that are also specific for c-erbB-2 protein if they bind c-erbB-2 protein alone or if, when properly incorporated into immunoglobulin conformation with complementary variable regions and constant regions as appropriate, are then capable of binding c-erbB-2 protein with specificity.

In competition assays the ability of an antibody or peptide fragment to bind an antigen can be determined by detecting the ability of the peptide to compete with the binding of a compound known to bind the antigen. Numerous types of competitive assays are known and are discussed herein. Alternatively, assays that measure binding of a test compound in the absence of an inhibitor may also be used. For instance, the ability of a molecule or other compound to bind the c-erbB-2 protein can be detected by labelling the molecule of interest directly or it may be unlabeled and detected indirectly using various sandwich assay formats. Numerous types of binding assays such as competitive binding assays are known (*see*, *e.g.*, U.S. Patent Nos. 3,376,110, 4,016,043, Harlow and Lane, *Antibodies: A Laboratory Manual*, Cold Spring Harbor Publications, N.Y. (1988), and Coligan, *et al.* (eds), *Current Protocol in Immunology,* Wiley and Sons, N. Y.

Assays for measuring binding of a test compound to one component alone rather than using a competition assay are also available. For instance, immunoglobulins can be used to identify the presence of the c-erbB-2 protein. Standard procedures for monoclonal antibody assays, such as ELISA, may be used (see, Harlow and Lane, *supra).* For a review of various signal producing systems which may be used, *see,* U.S. Patent No. 4,391,904.

Further, the specificity of the binding moieties to c-erbB-2 can be determined by their affinity. Such specificity exists if the dissociation constant ($K_D$ = 1/K, where K is the affinity constant) of the moiety is < 1µM, preferably < 100 nM, and most preferably < 1 nM. Antibody molecules will typically have a $K_D$ in the lower ranges. $K_D$ = [R-L]/[R][L] where [R], [L], and [R-L] are the concentrations at equilibrium of the receptor or c-erbB-2 (R), ligand, antibody, or peptide (L) and receptor-ligand complex (R-L), respectively. Typically, the binding interactions between ligand or peptide and receptor or antigen include reversible noncovalent associations such as electrostatic attraction, Van der Waals forces, and hydrogen bonds.

Other assay formats may involve the detection of the presence or absence of various physiological or chemical changes that result from the interaction, such as down modulation, internalization, or an increase in phosphorylation, as described in United States Patent Application No. 07/644,361 filed 1/18/91.

*See also, Receptor-Effector Coupling - A Practical Approach*, ed. Hulme, IRL Press, Oxford (1990).

A preferred peptide specific for c-erbB-2 protein induces an increase in the phosphorylation of the c-erbB-2 protein when placed in contact with tumor cells expressing the c-erbB-2 protein. A molecule that "induces an increase in the phosphorylation of c-erbB-2 protein" is one that causes a detectable increase in the incorporation of phosphate into the protein over that which occurs in the absence of the molecule. Typically this detectable increase will be a two-fold or greater increase in phosphorylation, preferably greater than a three-fold increase over controls. Phosphorylation may be measured by those methods known in the art for detecting phosphorylation of receptors. *See*, for example Cooper, *et* al., *Methods in Enzymology,* 99:387-402 (1983); Antoniades and Pantazis, *Methods in Enzymology,* 147: 36-40 (1987); and Lesniak, *et al.*, *Methods in Enzymology,* 150:717-723 (1987).

Typically, phosphorylation can be measured by *in vivo* phosphorylation of intact cells (Lesniak, *supra)* or by an *in vitro* autophosphorylation reaction (Antonaides, *supra*). For measuring *in vivo* phosphorylation, for example, assays may be conducted where cells bearing the c-erbB-2 protein are placed into contact with radioactive labeled phosphate. To detect phosphorylation of the c-erbB-2 protein receptor in the *in vivo* assay, it is advantageous to incubate the test cells for about 12 to about 18 hours, with the labeled phosphate. The cells are divided into two or more batches, where some are exposed to the molecule expected to increase the phosphorylation of the receptor and some are separated out as controls. The aliquots are subsequently immunoprecipitated, the receptor is recognized, for example, by SDS polyacrylamide gel or autoradiography methods, and an increase in phosphorylation is considered statistically significant when there is a two-fold or greater increase in the background of the aliquot exposed to the test molecule over the control aliquots.

To measure *in vitro* autophosphorylation, for example, cells or cell extracts may be incubated in the presence or absence of the peptide specific for c-erbB-2. Following immunoprecipitation with an anti-c-erbB-2 antibody, the immune complex may be incubated with $\gamma^{32}$P-ATP and analyzed by SDS-PAGE autoradiography.

Another preferred peptide specific for c-erbB-2 protein is one that causes down modulation of the c-erbB-2 protein. "Down modulation of the c-erbB-2 protein" is determined by a detectable decrease in the presence on the tumor cells of the c-erbB-2 receptor. Such down modulation is detected by a decrease in the ability of antibodies or other specific binding moieties to bind to or recognize the c-erbB-2 receptor protein on the tumor cells. For example, down modulation can be determined by incubating tumor cells bearing the c-erbB-2 protein receptor with the peptide of interest, washing the cells, then contacting the cells with labeled (preferably radiolabeled) antibodies specific for the c-erbB-2 protein. The extent of binding of the labeled anti-c-erbB-2 antibodies to the cells exposed to the peptide specific for c-erbB-2 protein is compared to the extent of binding of the antibodies to control cells (i.e., not exposed to the c-erbB-2 specific peptide). Preferably for these assays, the cells are directly subjected to the labeled anti-c-erbB-2 antibodies after washing.

The down modulation observed is typically dose dependent, i.e., the extent of down modulation increases with the amount of peptide specific for c-erbB-2 protein exposed to the c-erbB-2 protein. A peptide that causes a decrease in 90% or greater of binding of the treated cells versus control cells to anti-c-erbB-2 antibodies is preferred.

Another preferred peptide specific for c-erbB-2 protein is one that binds tumor cells expressing c-erbB-2 protein and is internalized when placed in contact with such tumor cells. "Internalization" occurs when the peptide becomes sequestered in the cytoplasm of the cells. Once internalized, the receptor and/or peptide may be degraded in the cell lysosomes or may be recycled to the cell surface. A method for determining internalization of a ligand-receptor complex is also described in Haigler, *et al.*, *J. Biol. Chem.,* 255:1239-1241 (1980).

A cell growth modulator is a molecule which affects the growth of a cell to which it is targeted. Typically, the modulator must be internalized into the target cell, but this function is usually provided by internalization which results from the targeting moiety.

The modulation will typically be a decrease in metabolism or growth rate, preferably a toxic effect, but a significant increase in metabolism or growth rate will also be useful. When a significant increase in metabolism or growth rate is effected, a short term poison might be used in combination to kill only those cells exhibiting such.

For modulators which decrease metabolism or growth rate, it is preferred that the modulator be highly potent, *e.g.*, have a very high activity.

Although viral and fungal toxins exist, particular bacterial or plant toxins have among the highest specific activities known. Growth arrest may occur by preventing any of a number of essential cellular functions including nucleic acid synthetic, protein synthesis, and cellular metabolism, general or specific. For example, pseudomonas exotoxin and diphtheria toxin function by irreversibly arresting protein synthesis in eukaryotic cells. Both examples enzymatically inactivate elongation factor 2, which is an essential component of protein synthesis. other elongation factors may be targets for other toxins. Ricin, in contrast, is a plant toxin which acts directly on the ribosome, acting on the 285 rRNA.

Preferably, the growth modulators will have enzymatic activities with high turnover numbers so internalization of very few molecules can kill the target cell: *See*, Pastain, *et al.*, *Science* 254:1173-1177.

Gelonin is a glycoprotein (M.W. approximately 29-30,000 Kd) purified from the seeds of *Gelonium multiforum* and belongs to a class of potent ribosomal-inactivating plant toxins. other members of this class include chains of abrin, ricin, and modeccin. Gelonin, like abrin and ricin, inhibits protein synthesis by damaging the 60S subunit of mammalian ribosomes. Gelonin appears to be stable to chemical and physical treatment. Furthermore, gelonin itself does not bind to cells and is normally non-toxic (except in high concentrations) when administered alone, and is safe to manipulate in the laboratory. The inactivation of ribosomes is irreversible, does not appear to involve co-factors, and occurs with an efficiency which suggests that gelonin acts enzymatically.

Gelonin and ricin inhibit protein synthesis and are among the most active toxins on a protein weight basis. Gelonin is 10 to 1000 times more active in inhibiting protein synthesis than ricin A chain. Peptides like ricin and abrin are composed of two chains, an A chain which is the toxic unit and a B chain which acts by binding to cells. Unlike ricin and abrin, gelonin is composed of a single chain, and, because it lacks a B chain for binding to cells, it is itself relatively inert, or non-toxic to intact cells. This feature of having a much lower cellular effect when not conjugated to a binding or targeting moiety is an important feature of various embodiments of the present invention. This differential toxicity is important in high specificity for c-erbB-2 expressing cells.

Mammalian cells apparently lack the ability to bind and/or to internalize the native gelonin molecule. Conjugates of gelonin with a tumor-targeting reagent, such as the monoclonal antibody TAb 250 directed to a tumor associated antigen present on certain tumor cells, provide both a specific method for binding the gelonin to the cell and a route for internalization of the gelonin-antibody complex.

The toxic moiety of the immunotoxin may be a toxic drug or an enzymatically active toxin of plant origin, or an enzymatically active fragment ("A chain") of such a toxin.

The active toxins can function by any of a number of mechanisms, each of which affects cellular physiology and growth. The toxins may be metabolic inhibitors or poisons, nucleic acid synthesis inhibitors, protein synthesis inhibitors, or any other mediators of abnormal or deleterious functions. Most preferred is conjugation with gelonin.

Active fragments and derivatives include any compounds which have the.same core structure as the full length structure of gelonin but lack the entire primary sequence. These fragments or derivatives will have the same or improved biological or toxic activity as gelonin. The toxicity of the gelonin fragments or derivatives may be routinely determined by those with skill in the art using the rabbit reticulocyte lysate assay.

The targeting moiety and the cell growth modulator may be conjugated using a variety of bifunctional protein coupling agents. Examples of such reagents are N-succinimidyl 3-(2-pyridyldithio)(propionate)(SPDP), 2-IT, 4-succinimidyloxycarbonyl-$\alpha$-methyl-$\alpha$(2-pyridyldithio)toluene (SMPT) bifunctional derivatives of imidoesters such as dimethyl adipimidate, HCl, active esters such as disuccinimidyl suberate, aldehydes such as glutaraldehyde, bis-azido compounds such as bis(p-azidobenzoyl) hexanediamine, bis-diazonium derivatives such as bis-(p-diazoniumbenzoyl)-ethylenediamine, diisocyanates such as tolylene 2,6-diisocyanate, and.bis-active fluorine compounds such as a 1,5-difluoro-2,4-dinitrobenzene.

Prior to use in these studies, the Sp2/0-Ag14 cells will be grown initially in the presence of 0.1 $\mu$g/ml of native gelonin. Over several months, the concentration of gelonin will be gradually increased until the cells can be maintained in up to 10 mg/ml. Cells will then be cloned by limiting dilution in the presence of 10 mg/ml gelonin and the resulting colonies resistant to gelonin will be expanded. Gelonin will then be removed from the culture media for two passages and the cells challenged again with gelonin exposure to confirm development of stably-resistant clones. After tests to confirm the production and activity of chimeric TAb-250, gelonin-resistant SP2/0 cell producing antibody will be grown and the cDNA for the TAb 250 antibody removed from the total DNA by incubation with restriction endonuclease. In parallel, the cDNA from JM105 E-Coli expressing optimized gelonin will be removed, purified and the DNA encoding gelonin released after digestion with HindIII and Eco RI. The gelonin gene will be ligated into the heavy-chain fragment and the insert replaced into gelonin resistant SP2/0 cells. Cells will then be sub-cloned by limiting dilution and the clones screened for both chimeric antibody production and gelonin content. Finally, positive clones will be expanded and the recombinant fusion protein will be purified and tested in both in vitro cytotoxicity assays and *in vivo* tissue distribution, pharmacokinetics, therapeutics and toxicity trials. A comparison of TAb-250-gelonin fusion protein properties to the characteristics of the previously described TAb 250 gelonin constructs will be performed to determine the advantages and drawbacks of each. Based upon these studies a Phase I clinical study of chimeric TAb 250 gelonin fusion protein may be performed in patients with advanced breast cancer.

Administration of the immunotoxins of the present invention to an individual who has been diagnosed as having neoplastic cells, e.g., a tumor with an undesirable level of expression of the c-erbB-2 oncogene, will allow targeting and concentration of the cytotoxic agent at the site where it is needed to kill them. By so targeting the toxic agents, non-specific toxicity to other organs, tissues and cells will be eliminated, minimized or at least decreased.

When used *in vivo* for therapy, the immunotoxins of the present invention are administered to the patient or an animal in therapeutically effective amounts, i.e., amounts that eliminate or reduce the tumor burden. They will normally be administered parenterally, preferably intravenously, but other routes of administration will be used as appropriate. The dose and dosage regimen will depend upon the nature of the cancer (primary or metastatic) and its population, the characteristics of the particular immunotoxin, *e.g.*, its therapeutic index, the patient, the patient's history and other factors. The amount of immunotoxin administered will typically be in the range of about 0.1 to about 10 mg/kg of patient weight. The schedule will be continued to optimize effectiveness while balanced against negative effects of treatment. See Remington's Pharmaceutical Science, 17th Ed. (1990) Mark Publishing Co., Easton, Penn.; *and Goodman and Gilman's: The Pharmacological Basis of Therapeutics* 8th Ed (1990) Pergamon Press.

For parenteral administration the immunotoxins will most typically be formulated in a unit dosage injectable form (solution, suspension, emulsion) in association with a pharmaceutically acceptable parenteral vehicle. Such vehicles are preferably non-toxic and non-therapeutic. Examples of such vehicles are water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances that enhance isotonicity and chemical stability, *e.g.*, buffers and preservatives. The immunotoxin will typically be formulated in such vehicles at concentrations of about 0.1 mg ml to 10 mg ml.

The immunotoxins of the present invention may also be used in an *in vitro* method. For example, the method may be used in killing tumor cells from bone marrow. In this method, the bone marrow is first removed from an individual having a neoplastic disease. Subsequently, the bone marrow is treated with a cytocidally effective dose of an immunotoxin of the present invention to eliminate the residual tumor cells. The treated bone marrow cells can be readministered to the patient to reestablish an immune system after receiving intensive chemotherapy and/or radiotherapy to eliminate all endogenous neoplastic hemototoxic cells.

The immunotoxins of the present invention may also be used to extend the survival time of tumor bearing mammals and to retard the rate of growth of tumors comprised of cancer cells carried by a mammal. For example, nude mice bearing xenografts of human tumors growing subcutaneously or intraperitoneally can be treated with doses of immunotoxin, antibody alone, toxin alone or saline at a dose between 25 and 100 mg/kg. Tumor growth inhibition can be measured by the change in physical size of the subcutaneous tumors or by prolongation of survival in mice-bearing intraperitoneally tumors such as SKOV-3 cells. Such studies may be useful or indicative of methodologies and which might be applicable to other mammals, including primates.

The following examples provide a detailed description of the preparation, characterization, and use of the immunotoxins of this invention. These examples are not intended to limit the invention in any manner.

Example 1

Purification of Gelonin

A procedure for the isolation of gelonin is available in Stirpe, *et al., I. Biol. Chem.,* 255, 6947-53 (1980). Seeds of *Gelonium multiflorum* were shelled and the nuts ground in a homogenizer with eight volumes of 0.14 M NaCl containing a 5 mM sodium phosphate (pH 7.4). The homogenate was left overnight at 4°C with continuous stirring, cooled on ice and centrifuged at 35,000 times g for 20 minutes at 0°C. The supernatant was removed, dialyzed against 5 mM sodium phosphate (pH 6.5) and concentrated using a pm10 filter. The sample was layered on a CM-52 ion-exchange column (20 x 1.5 cm) equilibrated with 5 mM sodium phosphate (pH 6.5). Material which bound to the ion exchange resin was eluted with 400 ml of a linear NaCl gradient from 0 to 0.3 M at a rate of 25 ml per hour at 4°C. Five ml fractions were collected. The fractions were monitored at 280 nm in a spectrophotometer. The gelonin eluted in about fractions 55-70 and was the last major elution peak. Fractions 55-70 were pooled, dialyzed against double distilled water and concentrated by lyophilization. The purity and the molecular weight of each preparation was checked on high pressure liquid chromatography using a TSK 3000 gel permeation column with 50 mM sodium phosphate buffer, pH 7.4 and 15% sodium dodecylsulphate-polyacrylamide gel electrophoresis (SDS-page). Gelonin migrated as a single band with an approximate molecular weight of 29-30,000 daltons.

Example 2

Assay of Gelonin Activity

The gelonin activity was monitored in a cell-free protein synthesis inhibition assay. The cell-free protein synthesis inhibition assay was performed by sequentially adding to 50 μl rabbit reticulocyte lysate, mixing after each addition, the following components: 0.5 ml of 0.2 M Tris-HCl (pH 7.8), 8.9 ml of ethylene glycol, and 0.25 ml of 1 M HCl).

Twenty microliters of a salt-amino acid-energy mixture (SAEM) consisting of: 0.375 M KCl, 10 mM Mg $(CH_3CO_2)_2$, 15 mM glucose, 0.25-10 mM amino acids (excluding leucine); 5 mM ATP, 1 mM GTP, 50 mM Tris-HCl (pH 7.6), 10 μl Creatinine phosphate-creatinine phosphokinase, 12 μl [3H]leucine (Amersham, 74 mci/mmol), and adding 1.5 μl of solutions containing varying concentrations of the gelonin mixture. The mixture was incubated for 60 minutes at 30°C. [3H] leucine incorporation was monitored in an aliquot of the mixture by precipitating synthesized protein on glass fiber filters, washing in 10% TCA and acetone, and monitoring the radioactivity in a Beta-counter using Aquasol scintillation fluid. Gelonin with a specific activity no lower than 4 x $10^9$ U/mg was used for conjugation with the antibodies. A unit of gelonin activity is the amount of gelonin protein which causes 50% inhibition of incorporation of [14C] leucine into protein in the cell free assay.

Example 3

Conjugation of Tab 250 With Gelonin Preparation of 2-IT Modified Gelonin

Gelonin in phosphate buffered saline was concentrated to approximately 10 mg/ml in a Centriprep 10 concentrator. Triethanolamine hydrochloride (TEA/HCl), pH 8.0, and EDTA were added to a final concentration of 60 mM TEA/HCl and 1 mM EDTA, pH 8.0. A 2-iminothiolane stock solution (500 mM in 60 mM TEA/HCl buffer containing 1 mM EDTA, pH 8.0) was added to a final concentration of 1 mM and the sample was incubated for 90 min at 4°C under a stream of nitrogen gas with stirring. Excess iminothiolane was removed by gel filtration on a column of Sephadex G-25 (1 × 24 cm) pre-equilibrated with phosphate-EDTA buffer, pH 7.5, containing 0.01 M $Na_2HPO_4$, 0.0018 M $KH_2PO_4$, 0.0034 M KCl, 0.001 M EDTA and 0.17 M NaCl. Fractions were analyzed for protein content in microtiter plates using Bio-Rad assay. Gelonin eluted at the void volume (about fractions 21-23). These fractions were pooled and stored at 4°C.

Example 4

Preparation of Monoclonal Antibodies

BALB/c mice were immunized intraperitoneally (i.p.) and subcutaneously (s.c.) with 2 x $10^6$-1 x $10^7$ NIH3T3$_T$ (NIH373 cells transfected with c-erbB-2) cells emulsified 1:1 in complete Freund's adjuvant. Animals were boosted every 2 to 4 weeks. When positive titers in an ELISA (described below) were detected, a final i.p. or intravenous (i.v.) boost was given 4 days before fusion. Spleen cells were fused with P3-X63Ag8.653 myeloma cells maintained in RPMI 1640, 10% FBS, and 2 mM L-glutamine. Hybridoma supernatants were tested for positive reactivity in an ELISA (see below), and extracellular domain reactivity was determined by indirect immunofluorescence using unfixed NIH3T3 and NIH3T3$_T$ cells at 4°C followed by flow cytometric analysis. The monoclonal antibody TAb 250 may be obtained from any source. Most preferably, the anti-c-erbB-2 antibody used in the present invention is either a human antibody or a murine antibody.

Hybridoma cells producing TAb 250 are grown in a 2L continuous perfusion bioreactor. The cell supernatant from the bioreactor is filtered and then passed through a Protein-G Trio system followed by ion exchange chromatography. The material is then concentrated and sterile filtered. Testing of final product includes tests for total DNA, protein purity, pH, (IEF), total protein, endotoxin, potency, identity and protein-G antigen.

The present invention, *inter alia*, utilizes a chimeric antibody, including a hybrid antibody or a humanized or human-like antibody. In a preferred embodiment, the variable sequence originates from and is substantially identical to a sequence of the murine TAb 250 antibody. Such a chimeric antibody is BACh-250.

Example 5

ELISA Assay

Sterile 96-well plates were pretreated for 2 hours at 37°C with bovine collagen at 1 mg/ml in sterile PBS. NIH3T3$_T$ (NIH353 cells transformed with the vector) cells were grown to 80% confluence and harvested with warm Puck's Versene (0.02% EDTA in PBS), washed, and plated at 0.5-1 x $10^6$ cells/ml in the treated wells overnight at 37°C. Plates were gently washed and treated with 10% neutral buffered formalin followed by a blocking step with 1% bovine serum albumin BSA/PBS. Sample supernatants or antibody dilutions were then added to the plates and incubated for 2 hours at 37°C followed by incubation with an alkaline phosphatase-conjugated goat anti-mouse IgG Fc-specific secondary antibody and incubated for 1 hour at 37°C. Plates were washed with PBS, a para-nitrophenyl phosphate and diethanolamine substrate were added and incubated for 15 minutes at room temperature, and $A_{405}$ was measured. Supernatants or antibodies that reacted with the transfected cells at an absorbance of 0.2-1.0 greater than the absorbance for a negative control antibody were considered positive.

Example 6

Preparation and Handling of $^{125}$I-TAb 250

TAb 250 was radiolabeled using Iodobeads (Pierce) according to the manufacturer's specifications. Carrier-free Na$^{125}$I (400 uCi of IMS. 30, Amersham) was reacted with 25 ug TAb 250 in 100 mM Na-phosphate buffer (200 μl, pH 7.4) in the presence of 3 Iodobeads. This resulted in an approximate ratio of one iodine atom per IgG molecule. The incorporation was allowed to proceed at room temperature for 7.5 minutes with intermittent mixing. The reaction mixture was removed from the beads, and after 5 minutes, the volume was adjusted to 0.5 ml with Na-phosphate buffer and 2 μl were taken to estimate specific activity (see below). The remaining volume was desalted by gel filtration using a NAP-5 column (Pharmacia) equilibrated with PBS containing 0.1% BSA and 0.02% azide. The radiolabeled antibody was eluted in 1 ml column buffer and was stored at 4°C for up to six weeks with no apparent loss of binding activity. The desalted material was essentially free of unincorporated iodine since >95% was TCA-precipitable.

The specific activity of the radiolabeled antibody was estimated by TCA precipitation of the material before the de-salting step. Thus, 2 μl of the reaction mixture was diluted 500-fold in column buffer and duplicate aliquots mixed with an equal volume of ice-cold 20% TCA. After 25 minutes on ice the precipitated material was collected by centrifugation (10 min, 3000 xg). Supernatants and pellets were counted separately, and the incorporation was expressed as the percent of TCA-precipitable counts. The incorporation obtained in separate iodinations ranged from 27% to 45%, yielding specific activity estimates from 3.9 to 7.2 uCi/ug. Before each binding experiment, an appropriate amount of $^{125}$I-TAb 250 was de-salted by gel filtration using a NAP-5 column equilibrated in binding buffer. This procedure removed the azide and yielded material that was routinely >98% TCA-precipitable.

Example 7

Cell Culture

The human breast adenocarcinoma cell lines, SKBR-3, MDA-MB-453, and MDA-MB-231, and the human ovarian adenocarcinoma cell line SKOV-3, were used. SKBR-3, MDA-MB-231, MDA-MB-453 cells were maintained in minimal essential medium supplemented with 10% FBS and 2 mM L-glutamine. Medium for MDA-MB-453 cells also contained 1% non-essential amino acid and 1% vitamins. SKOV-3 cells were cultured in Iscove's modified Dulbecco's medium supplemented with 10% FBS and 2 mM L-glutamine. All cultures were incubated at 37°C in either 5 or 10% $CO_2$ as required.

Example 8

Internalization of $^{125}$I-TAb 250

Internalization of $^{125}$I-TAb 250 was assessed by determining the amount of radioactivity in acid sensitive and insensitive compartments. Cells were harvested and resuspended in ice cold binding buffer with $^{125}$I-TAb 250 alone (from 6 ng/ml to 153 ng/ml) or with excess unlabeled TAb 250 to determine non-specific binding. After the cell surface binding of the radiolabeled antibody reached equilibrium, the cells were pelleted at 200 x g for 5 minutes 4° C and washed three times with ice cold binding buffer to remove unbound antibody. The cell pellets were resuspended in ice cold binding medium, and aliquots were taken to determine the amount of initial $^{125}$I-TAb 250 surface binding. To initiate internalization of the radiolabeled antibody, the cells were warmed to 37° C. At the times from 15 to 150 minutes, aliquots were removed and the cells collected by centrifugation (1400 x g 5 minutes, 4°C). The supernatants which contained dissociated or recycled antibody were collected. The pellets were resuspended twice in an acid wash (100 μl/tube PBS, 1% glucose, pH 1). Supernatants containing the surface-bound antibody were combined and counted. The tips of the tubes containing the remaining cell associated radioactivity were clipped and counted.

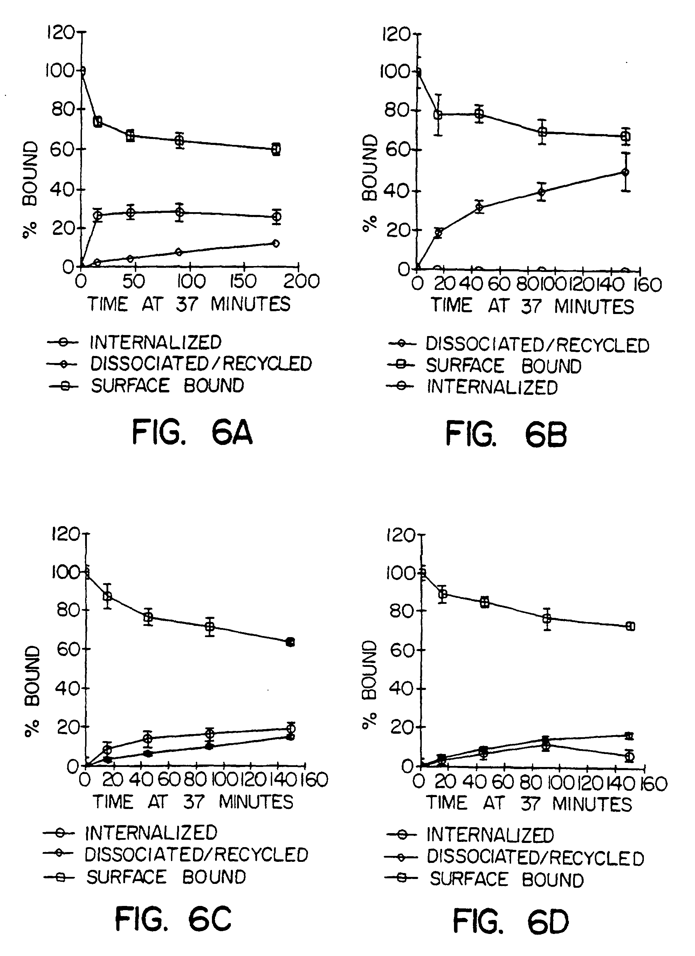

Figure 6 illustrates that monoclonal antibody TAb 250 is not internalized in MDA-MB-231 cells (panel B). In contrast, SKBR-3 cells internalized the TAb 250 antibody most efficiently (panel A) while internalization of the antibody into SKOV-3 cells and MDA-MB-453 cells was intermediate (panels C and D, respectively).

Example 9

Modification of Monoclonal Antibody TAb 250 With SPDP

N-succinimidyl 3-(2-pyridyldithio)(propionate)(SPDP) in dimethylformamide was prepared as a stock solution of 3 mg ml in dry dimethylformamide. Since the crystalline SPDP can undergo hydrolysis, the actual concentration of chemically reactive crosslinker was determined by spectrophotometric methods by analyzing the absorbance at 260 nm in a dual-beam spectrophotometer. The concentration of SPDP stock is calculated from the following equation:

$$\frac{\text{Change in absorbance (260 nm)}}{0.02 \times 103 \text{ ml mmol}} \times \frac{(301)}{0.01} = \text{mmoles/ml/SPDP}$$

One milligram of monoclonal antibody TAb 250 in 1.0 ml of phosphate buffered saline (PBS) was added to a glass tube. SPDP stock solution was slowly added at about a 5-fold molar excess to the tube (approximately 10 μl of

stock solution), mixing constantly. The mixture was incubated for 30 minutes at room temperature, mixing every 5 minutes during the incubation period.

Excess unreacted SPDP was removed from the sample by gel filtration chromatography on a Sephdex G-25 column (1 x 24 cm) pre-equilibrated with 100 mM sodium phosphate buffer pH 7.0 containing 0.5 mM EDTA (Buffer A). Fractions (0.5 ml) were collected and analyzed for protein content using the Bradford dye binding assay (Bradford, Anal. Biochem. 72: 248-254 (1976). Absorbance (600 nm) was monitored in a 96-well plate using a Bio-TEK Microplate autoreader. Antibody eluted at the void volume (fractions 14-20) and these fractions were pooled and kept at 4°C. The protein was concentrated in a Centricon-30 microconcentrator. The Centricon retentate was washed with 100 mM sodium phosphate buffer, pH 7.0 containing EDTA (0.5 mM). The antibody was concentrated to a final volume of approximately 0.5-0.75 ml.

Example 10

Conjugation of SPDP-Modified Monoclonal Antibody TAb 250 With Iminothiolane-modified Gelonin

Conjugation of 2-IT Modified Gelonin and TAb 250

TAb 250-gelonin linked with SMPT is prepared by coupling 2-IT-modified gelonin with SMPT-modified monoclonal antibody TAb 250. Briefly, to modify TAb 250 with SMPT, 10 mg of antibody in 1.0 ml of PBS is diluted 1:1 with 2X borate buffer (0.05 M sodium borate 1.7% sodium chloride, pH 9.0) and 52 $\mu$l of 4 mM SMPT in dry DMF is slowly added to the antibody solution. The reaction is incubated at room temperature for 2 hr with stirring under $N_2$. Excess SMPT is removed by passing the reactions mixture through a Sephadex G-25 column containing phosphate-EDTA buffer, pH 7.5, and antibody positive fractions,are evaluated by Bio-Rad assay. The fractions are pooled and stored at 4°C under $N_2$. The cross-link with 2-IT is carried out at 27°C under $N_2$ with stirring for 96 hr. The final product is purified as described for SPDP in Example 9.

One milligram of purified gelonin (2 mg/ml in PBS) prepared as described in Example 1 was modified with iminothiolane as described in Example 3. Monoclonal antibody TAb 250 modified as described in Example 9 was mixed with an equal weight of the modified gelonin. This proportion corresponded to a 5-fold molar excess of gelonin as compared to antibody. The pH of the mixture was adjusted to 7.0 by the addition of 0.05 M TEA/HCl buffer pH 8.0 and the mixture was incubated for 20 hours at 4°C under nitrogen. Iodoacetamide (0.1 M) was added to a final concentration of 2 mM to block any remaining free sulfhydryl groups and incubation was continued for an additional hour at about 25°C. The reaction mixture was stored at 4°C until purification by gel filtration.

Example 11

Purification of Gelonin-Monoclonal Antibody TAb 250 Complexes

Non-conjugated gelonin and low molecular weight products were removed from the reaction mixtures of Example 10 by gel filtration on a Sephadex S-300 column (1.6 x 31 cm) pre-equilibrated with PBS.

Reaction mixtures from Example 10 were concentrated to approximately 1 ml with a Centricon 30 microconcentrator before loading on the Sephadex column. The column was washed with PBS. One ml fractions were collected and 50 $\mu$l aliquots are analyzed for protein by the Bradford assay.

Non-conjugated antibody was removed from the gelonin conjugated antibody by affinity chromatography on a column (1 x 24 cm) of Blue Sepharose CL-6B pre-equilibrated with 10 mM phosphate buffer, pH 7.2 containing 0.1 M NaCl. After loading the S-300 eluate sample, the column was washed with 30 ml of the same buffer to completely elute non-conjugated antibody.

Gelonin-conjugated antibody bound to the column and was eluted with a linear salt gradient of 0.2 to 2 M NaCl in 10 mM phosphate buffer, pH 7.2. The antibody-gelonin complex eluted at approximately 0.7 M NaCl. Protein content of the eluted fractions was determined by the Bradford assay. The protein-containing fractions were pooled and the elution pattern confirmed by electrophoresis on a 5 to 20% gradient non-reducing polyacrylamide gel. The flow-through peak (fractions 14-20) contains only free antibody while fractions 50-80, eluted with high salt, contain TAb 250-gelonin conjugate free of unconjugated gelonin or antibody. The final product contained TAb 250 antibody coupled to 1, 2 and 3 gelonin molecules. Average gelonin content was 1.5 molecules per antibody molecule. The rabbit reticulocyte *in vitro* translation system was utilized to estimate the gelonin activity of the essentially pure gelonin-TAB 250 antibody complex. One unit of activity in this assay was defined as the amount of protein required to provide 50% inhibition of protein synthesis as compared to untreated controls. Utilizing this assay, the specific activity of both the native gelonin and the TAb 250 gelonin conjugate were determined to be 2 x $10^8$ U/mg and 8.2 x $10^5$ U/mg, respectively. The essentially pure gelonin-TAb 250 antibody is active in the reticulocyte lysate assay. A 1:1000 dilution of the original sample caused approximately a 50% inhibition of protein synthesis, i.e., a 50% reduction of the incorporation of [$^{14}$C] leucine into protein. Thus, the activity of the original preparation was 1000 U/ml.

The compositions of the present invention may include fusion constructs of the TAb 250 monoclonal antibody and a cytotoxic moiety. Fusion constructs of the immunotoxin of the present invention may be prepared, *e.g.*, by the following method. The nucleotide sequence of both the H and L chain V regions of TAb 250 are easily determined. For example, total RNA is extracted from TAb 250 producing cells with quandinium thiocyanate. Poly A+ RNA can be isolated by oligo (dT) cellulose chromatography. The appropriate genes can be isolated using standard techniques, including reverse transcription and PCR techniques. A cDNA strand may be synthesized from isolated mRNA using an oligo-dT primer and reverse transcriptase. Such a cDNA strand can be amplified using standard PCR techniques with appropriate primers. A primer near the poly-A tail of the message can be based either upon the poly-A sequence, or upon common adjacent sequences found in mouse immunoglobulins. See Devereaux, Genetics Computer Group, University of Wisconsin Biotechnology Center and its associated sequence databases. A primer at the other end of the gene may be selected from common sequences found in mouse immunoglobulins. See Orlandi et al. (1989) *PNAS* 86:3833-3837 and Larrick et al. (1989) *Bio/Technology* 7:934-938. The TAb 250 heavy chain gene has a 5' upstream sequence of ATATAG CAGGAC CATATG and starts coding with ATGAA CTTGG GGCTC. The TAb 250 light chain gene has a 5' upstream sequence TTTAC TTCCT TATTT and starts coding with ATGGG CATCA AGATG. These primers can be used to amplify the genes by PCK technology, and cloned into plasmid expression vectors.

Transfection of DNA into Mouse Cells by Electroporation

Standard transfection methods can be applied to these genes. For example, DNA can be introduced into murine hybridoma Sp2/0-AG14 cells by electroporation. 1-2 x 10$^3$ actively growing SP2/0-AG14 cells are washed and resuspended in 1.0 ml of sterile PBS. Thirty micrograms of each chimeric, IgK and IgG1, plasmid is added to the cell suspension. The DNA/cells are transferred to a precooled shocking cuvette, incubated on ice at least 5 minutes and then a 0.5 kv/cm electro-pulse is delivered for 10 msec (Transfector 300, BTX). After shocking, the DNA/cell mixture is returned to ice for 10 minutes, diluted in 10 ml of DMEM containing 5% NCTC-109 and 10% FCS, and incubated at room temperature for 10 minutes. Finally, the cells are transferred to a 37°C incubator with 7% CO$_2$ for 48 hours before plating in selective medium, containing 1 μg/ml Xanthine. Cells can be plated in 96-well plates at 3x10$^4$ cells/well and the culture supernatants assayed by ELISA for antibody bound to TAb 250 antigen positive target cells.

Example 12

MTT Assay

3-(4,5-dimethyl/thiazolyl)-2,5-diphenyltetrazolium bromide (MTT) assays were carried out by removing cells from tissue culture flasks with versene 1:5000, centrifuging at 500 x g for five minutes, and resuspending the cells in medium at a concentration of 1 x 10$^5$ cell/ml. Cells were plated at 100 μl/well into 96-well microtiter plates and incubated in a humidified CO$_2$ incubator at 37° C for 24 hours.

On the next day, TAb 250 or TAb-250 gelonin was added. Immediately after deposition of the highest antibody concentration into the first column of wells, 1:2 dilutions of antibody were performed directly in the microtiter plates using a multichannel pipette. Plates were then incubated for three days, followed by the addition of 10 μl/well of MTT. MTT was prepared as a 5 mg/ml solution of PBS, filtered sterilized, and stored at 4° C in the dark. Plates were kept dark and incubated for an additional 4 hours at 37° C. The MTT crystals were dissolved by mixing the contents of the wells vigorously with 100 microliters of isopropanol containing 0.04 N HCl and 3% sodium dodecylsulfate. Absorbants at 570 nm was determined using an enzyme-linked immunosorbent assay (ELISA) reader.

Example 13

CPA

The cell proliferation assay (CPA) measures cell growth by determination of cell number and viability. On Day 0, cells at 80-90% confluency are released from a tissue culture flask, pelleted at 200 x g at 20°C for 6 minutes, and resuspended in Iscove's MEM containing 2 mM glutamine and 10% fetal bovine serum to a concentration of 6000 cells/ml. The cell suspension is added to 24-well plates at 1 ml per well. The plates are incubated at 37°C, 5% CO$_2$, for 24 hours. On day 1, the cells in three wells are washed with PBS and released from the plate with 1 ml 0.05% trypsin in PBS and 500 μl are counted using a Coulter Counter. To the remaining wells are added 20 μl of PBS, TAb 250, or TAb 250-gelonin, at the concentrations indicated in the figures. The plates are returned to the incubator. At the time-points indicated in the figures, the cells are released with trypsin and counted as on Day 1. The remaining 500 μl of cells are

stained with propidium iodide so that viability can be determined by flow cytometry. For each point on the graph, a mean cell number (n=3) is determined and multiplied by the percent of live cells to find the number of viable cells. This is divided by the number of live cells treated with PBS to the percent of control on the Y axis.

Example 14

Cytotoxicity of Gelonin and Gelonin-TAb 250 Antibody Complex

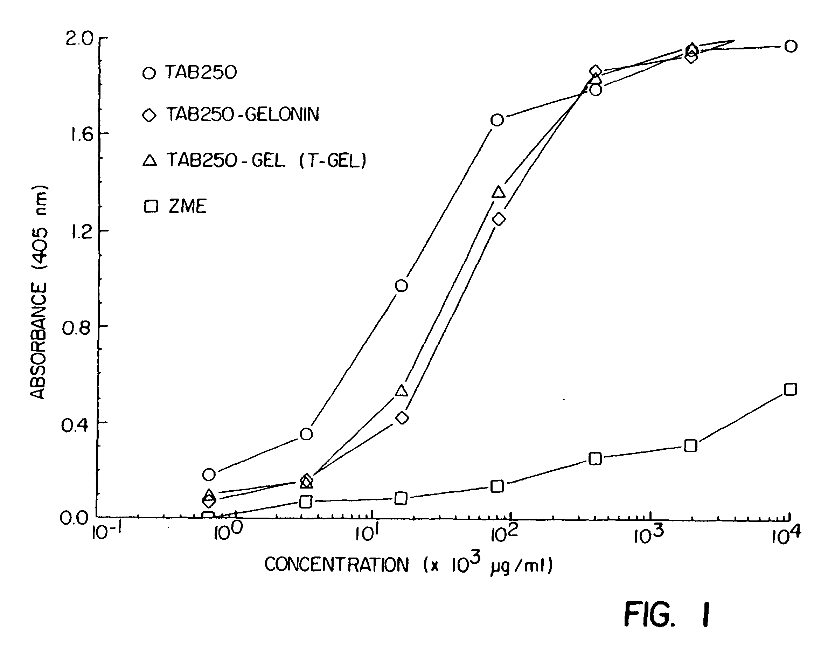

As can be seen in Figure 1, ZME antibody had virtually no effect on SKOV-3 cells. In contrast, TAb 250-gelonin immunoconjugate was highly active.

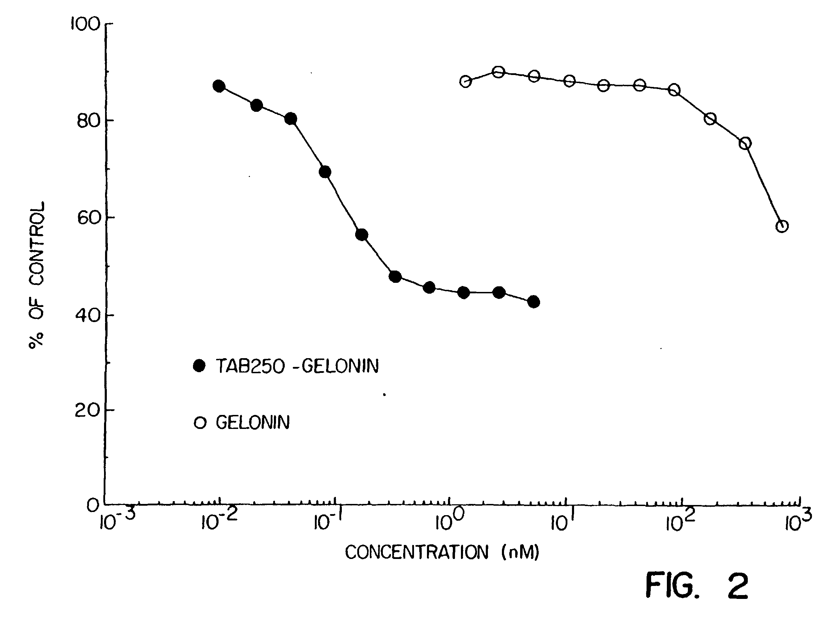

Figure 2 illustrates a cytotoxicity of the TAb 250-gelonin immunoconjugate on SKOV-3 cells compared to gelonin alone. At the same concentration, TAb 250-gelonin immunoconjugate was approximately 10,000 x as cytotoxic as gelonin alone.

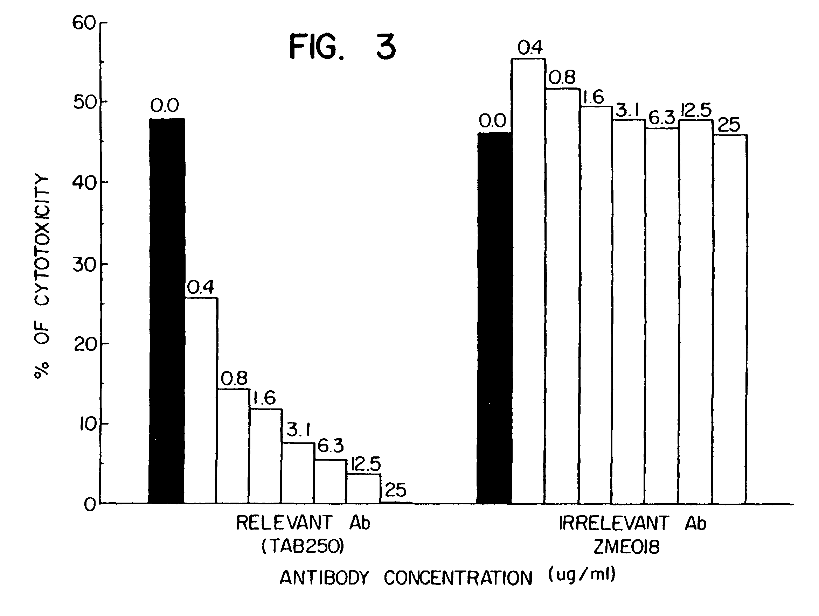

Figure 3 demonstrates that an irrelevant antibody, ZME 18 monoclonal antibody, has no competitive effect on the cytotoxicity of the TAb 250-gelonin immunoconjugate. In contrast, increasing concentration of TAb 250 monoclonal antibody decreases the cytotoxicity of the immunoconjugate in a dose dependent fashion.

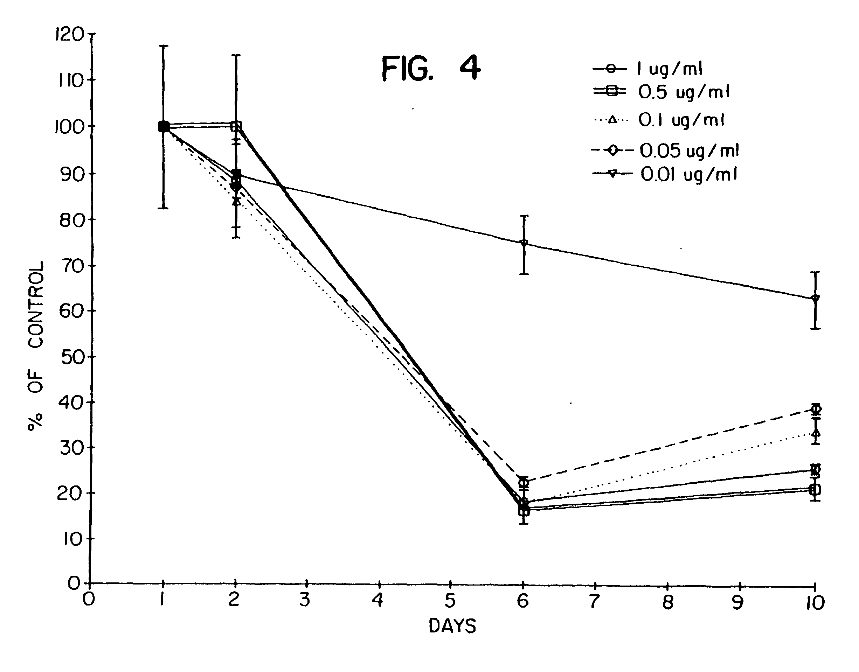

Figure 4 illustrates a dose response relationship of the TAb 250-gelonin immunoconjugate on SKOV-3 cells. As seen in the CPA assay, a dose above 0.1 micrograms/ml produced 80% inhibition at six days.

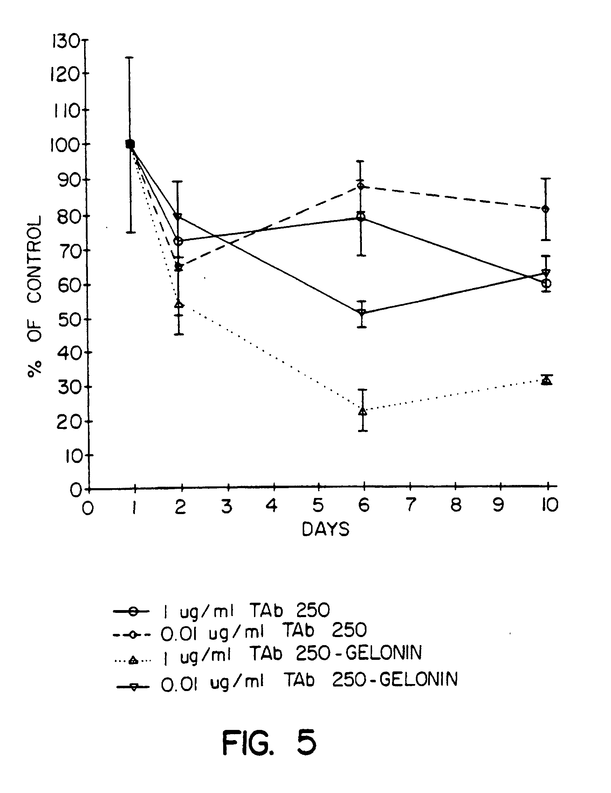

Figure 5 illustrates the effects of either the monoclonal antibody TAb 250 alone or the conjugate of TAb 250 with gelonin on SKOV-3 cells. As can be seen, there's a dose dependent inhibition by the TAb 250-gelonin immunoconjugate in the CPA assay.

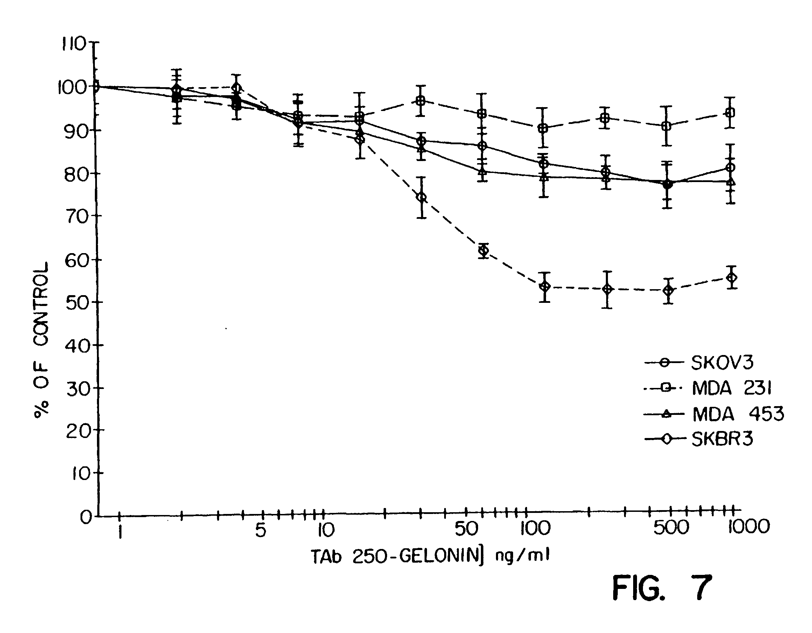

Figure 7 depicts the effects of the TAb 250-gelonin immunoconjugate on four different cell lines. As would be expected, the greatest amount of toxicity occurred in the SKBR-3 cell line. Intermediate toxicity was evidenced in SKOV-3 and MDA-MB-453 cells while virtually no cytotoxicity was seen in MDA-MB-231 cells. Thus, the cytotoxicity of the TAb 250-gelonin immunoconjugate correlates with the number of cell surface receptors in these cells.

In conclusion, therefore, it is seen that the present invention and the embodiments disclosed herein are well adapted to carry out the objectives and obtain the ends set forth at the outset.

International Application No: PCT/

## MICROORGANISMS

Optional Sheet in connection with the microorganism referred to on page __18__ line __3__ of the description [1]

**A. IDENTIFICATION OF DEPOSIT** [1]

Further deposits are identified on an additional sheet ☐ [1]

Name of depositary institution [1]

American Type Culture Collection (ATCC)

Address of depositary institution (including postal code and country) [1]

12301 Parklawn Drive
Rockville, Maryland  20852, United States of America

| Date of deposit [1] | Accession Number [1] |
|---|---|
| 16 January 1991 | HB 10646 |

**B. ADDITIONAL INDICATIONS** [1] (leave blank if not applicable). This information is continued on a separate attached sheet ☐

**C. DESIGNATED STATES FOR WHICH INDICATIONS ARE MADE** [1] (if the indications are not for all designated States)

**D. SEPARATE FURNISHING OF INDICATIONS** [1] (leave blank if not applicable)

The indications listed below will be submitted to the International Bureau later [1] (Specify the general nature of the indications e.g.
" Accession Number of Deposit ")

**E. ☐** This sheet was received with the international application when filed (to be checked by the receiving Office)

_Mamie Holmes_
(Authorized Officer)

☐ The date of receipt (from the applicant) by the International Bureau [1]

was _____
(Authorized Officer)

(January 1985)

Form PCT/RO/134 (January 1981)

17

**Claims**

1. A composition comprising a conjugate of a protein exhibiting binding specificity for an antigen domain for c-erbB-2 protein and a plant derived toxin, wherein said toxin is selected from the group consisting of gelonin and full length recombinant gelonin.

2. A composition according to claim 1, wherein said binding specificity is for an extracellular epitope of c-erbB-2.

3. A composition according to claim 1 or claim 2, wherein said protein is derived from a $V_H$ segment of an antibody.

4. A composition according to any one of claims 1 to 3, wherein said protein comprises an intact immunoglobulin heavy chain.

5. A composition according to any one of the preceding claims, wherein said protein is derived from a $V_L$ segment of an antibody.

6. A composition according to any one of the preceding claims, wherein said protein comprises an intact immunoglobulin light chain.

7. A composition according to any one of the preceding claims, wherein said protein further comprises an intact immunoglobulin heavy chain.

8. A composition according to any preceding claim, wherein said protein is from a single chain antibody.

9. A composition according to any preceding claim, wherein said protein is from TAb 250 (ATCC Acession No. HB 10646) or BACh-250 (Humanised TAb 250).

10. A composition according to any preceding claim, wherein said plant derived toxin has a much lower cellular effect when not conjugated to said targeting moiety.

11. A composition according to any preceding claim, wherein said toxin is selected from the group consisting of native toxin and recombinant toxin.

12. A composition according to any preceding claim, wherein said conjugate is a fusion protein between said targeting moiety and said plant derived toxin.

13. A composition according to any preceding claim, further comprising a pharmaceutically acceptable vehicle.

14. A pharmaceutical comprising a composition according to any preceding claim.

15. Use of a composition according to any preceding claim in the preparation of a medicament for the treatment of a neoplastic cell.

16. Use of a composition according to any one of claims 1 to 14 in the preparation of a medicament for the treatment of over-expression of c-erbB-2 protein.

17. Use of a composition according to claim 1 in the preparation of a medicament for the treatment of neoplastic cells wherein said cell is selected from the group consisting of a mammary carcinoma cell, an ovarian carcinoma cell, a lung carcinoma cell, a salivary gland carcinoma cell, a gastric tumor cell, a colon adenocarcinoma cell, and a bone marrow leukemia cell.

18. Use of a composition according to any one of claims 1 to 14 in the preparation of a medicament for retarding the rate of growth of neoplastic cells.

19. Use of a composition according to any one of claims 1 to 14 in the preparation of a medicament for the treatment of human or animal neoplastic cells.

20. Use of a composition according to any one of claims 1 to 14 in the preparation of a medicament for the prevention of recurrence of a neoplastic condition.

21. Use of a composition according to any one of claims 1 to 14 in the preparation of a medicament for extending the survival time of a host of said neoplastic cell.

22. An *in vitro* method of treating a neoplastic cell, the method comprising administering an effective dose of a composition according to any one of claims 1 to 14 to the cells.

23. A method according to claim 22, wherein said neoplastic cell is a bone marrow cell.

24. Use of a composition according to any one of claims 1 to 14 in the preparation of a medicament for killing neoplastic cells in bone marrow.

25. Use of a composition according to claim 24, wherein the bone marrow cells overexpress c-erbB-2 protein.


**Patentansprüche**

1. Zusammensetzung, die folgendes aufweist:

    ein Konjugat eines Proteins, das eine Bindungsspezifität für eine Antigendomäne für das Protein c-erbB-2 zeigt, und eines von einer Pflanze stammenden Toxins,

    wobei das Toxin aus der Gruppe ausgewählt ist, die aus Gelonin und rekombinantem Gelonin mit der gesamten Länge besteht.

2. Zusammensetzung nach Anspruch 1, wobei die Bindungsspezifität für ein extrazelluläres Epitop von c-erbB-2 gilt.

3. Zusammensetzung nach Anspruch 1 oder Anspruch 2, wobei das Protein von einem $V_H$-Segment eines Antikörpers stammt.

4. Zusammensetzung nach einem der Ansprüche 1 bis 3, wobei das Protein eine schwere Kette eines intakten Immunglobulins aufweist.

5. Zusammensetzung nach einem der vorstehenden Ansprüche, wobei das Protein von einem $V_L$-Segment eines Antikörpers stammt.

6. Zusammensetzung nach einem der vorstehenden Ansprüche, wobei das Protein eine leichte Kette eines intakten Immunglobulins aufweist.

7. Zusammensetzung nach einem der vorstehenden Ansprüche, wobei das Protein ferner eine schwere Kette eines intakten Immunglobulins aufweist.

8. Zusammensetzung nach einem der vorstehenden Ansprüche, wobei das Protein von einer einzigen Kette eines Antikörpers stammt.

9. Zusammensetzung nach einem der vorstehenden Ansprüche, wobei das Protein von TAb 250 (ATCC-Zugangsnummer HB 10646) oder BACh-250 (humanisiertes TAb 250) stammt.

10. Zusammensetzung nach einem der vorstehenden Ansprüche, wobei das von einer Pflanze stammende Toxin einen viel geringeren zellulären Effekt hat, wenn es nicht mit der Zieleinheit konjugiert ist.

11. Zusammensetzung nach einem der vorstehenden Ansprüche, wobei das Toxin aus der Gruppe ausgewählt ist, die aus natürlichem Toxin und rekombinantem Toxin besteht.

12. Zusammensetzung nach einem der vorstehenden Ansprüche, wobei das Konjugat ein Fusionsprotein zwischen der Zieleinheit und dem von einer Pflanze stammenden Toxin ist.

13. Zusammensetzung nach einem der vorstehenden Ansprüche, die ferner einen pharmazeutisch akzeptablen Träger aufweist.

14. Pharmazeutische Zusammensetzung, die eine Zusammensetzung nach einem der vorstehenden Ansprüche aufweist.

15. Verwendung einer Zusammensetzung nach einem der vorstehenden Ansprüche bei der Herstellung eines Medikamentes für die Behandlung einer neoplastischen Zelle.

16. Verwendung einer Zusammensetzung nach einem der Ansprüche 1 bis 14 bei der Herstellung eines Medikamentes für die Behandlung der übermäßigen Ausprägung des Proteins c-erbB-2.

17. Verwendung einer Zusammensetzung nach Anspruch 1 bei der Herstellung eines Medikamentes für die Behandlung von neoplastischen Zellen, wobei die Zelle aus der Gruppe ausgewählt ist, die aus einer Mammakarzinomzelle, einer Ovariumkarzinomzelle, einer Lungenkarzinomzelle, einer Speicheldrüsenkarzinomzelle, einer Magentumorzelle, einer Adenokarzinomzelle des Kolons und einer Leukämiezelle des Knochenmarks besteht.

18. Verwendung einer Zusammensetzung nach einem der Ansprüche 1 bis 14 bei der Herstellung eines Medikamentes für die Verzögerung der Wachstumsrate von neoplastischen Zellen.

19. Verwendung einer Zusammensetzung nach einem der Ansprüche 1 bis 14 bei der Herstellung eines Medikamentes für die Behandlung von neoplastischen Zellen von einem Menschen oder einem Tier.

20. Verwendung einer Zusammensetzung nach einem der Ansprüche 1 bis 14 bei der Herstellung eines Medikamentes für die Verhinderung des Wiederauftretens eines neoplastischen Zustandes.

21. Verwendung einer Zusammensetzung nach einem der Ansprüche 1 bis 14 bei der Herstellung eines Medikamentes für die Verlängerung der Überlebenszeit eines Wirtes der neoplastischen Zelle.

22. In vitro Verfahren zur Behandlung einer neoplastischen Zelle, wobei bei diesem Verfahren den Zellen eine wirksame Dosis einer Zusammensetzung nach einem der Ansprüche 1 bis 14 zugeführt wird.

23. Verfahren nach Anspruch 22, wobei die neoplastische Zelle eine Knochenmarkszelle ist.

24. Verwendung einer Zusammensetzung nach einem der Ansprüche 1 bis 14 bei der Herstellung eines Medikamentes für die Abtötung von neoplastischen Zellen im Knochenmark.

25. Verwendung einer Zusammensetzung nach Anspruch 24, wobei die Knochenmarkszelle das Protein c-erbB-2 übermäßig ausprägt.

**Revendications**

1. Composition comprenant un conjugué d'une protéine présentant une spécificité de fixation pour un domaine antigénique de la protéine c-erbB-2 et d'une toxine dérivée d'une plante, dans laquelle ladite toxine est choisie dans le groupe constitué par la gélonine et la gélonine entière recombinante.

2. Composition selon la revendication 1, dans laquelle ladite spécificité de fixation vise un épitope extracellulaire de c-erbB-2.

3. Composition selon la revendication 1 ou 2, dans laquelle ladite protéine est dérivée d'un segment $V_H$ d'un anticorps.

4. Composition selon l'une quelconque des revendications 1 à 3, dans laquelle ladite protéine comprend une chaîne lourde d'immunoglobuline intacte.

5. Composition selon l'une quelconque des revendications précédentes, dans laquelle ladite protéine est dérivée d'un segment $V_L$ d'un anticorps.

6. Composition selon l'une quelconque des revendications précédentes, dans laquelle ladite protéine comprend une chaîne légère d'immunoglobuline intacte.

7. Composition selon l'une quelconque des revendications précédentes, dans laquelle ladite protéine comprend en outre une chaîne lourde d'immunoglobuline intacte.

8. Composition selon l'une quelconque des revendications précédentes, dans laquelle ladite protéine provient d'un anticorps monocaténaire.

9. Composition selon l'une quelconque des revendications précédentes, dans laquelle ladite protéine provient de TAb 250 (n° d'accès ATCC HB 10646) ou de BACh-250 (TAb 250 humanisé).

10. Composition selon l'une quelconque des revendications précédentes, dans laquelle ladite toxine dérivée d'une plante a un effet cellulaire bien plus réduit lorsqu'elle n'est pas conjuguée à ladite fraction de ciblage.

11. Composition selon l'une quelconque des revendications précédentes, dans laquelle ladite toxine est choisie parmi le groupe constitué par la toxine native et la toxine recombinante.

12. Composition selon l'une quelconque des revendications précédentes, dans laquelle ledit conjugué est une protéine hybride entre ladite fraction de ciblage et ladite toxine dérivée d'une plante.

13. Composition selon l'une quelconque des revendications précédentes, comprenant en outre un excipient pharmaceutiquement acceptable.

14. Produit pharmaceutique comprenant une composition selon l'une quelconque des revendications précédentes.

15. Utilisation d'une composition selon l'une quelconque des revendications précédentes dans la préparation d'un médicament destiné à traiter une cellule néoplasique.

16. Utilisation d'une composition selon l'une quelconque des revendications 1 à 14 dans la préparation d'un médicament destiné à traiter la surexpression de la protéine c-erbB-2.

17. Utilisation d'une composition selon la revendication 1 dans la préparation d'un médicament destiné à traiter des cellules néoplasiques où ladite cellule est choisie dans le groupe constitué par une cellule de carcinome mammaire, une cellule de carcinome ovarien, une cellule de carcinome pulmonaire, une cellule de carcinome des glandes salivaires, une cellule tumorale gastrique, une cellule d'adénocarcinome du côlon et une cellule leucémique de moelle osseuse.

18. Utilisation d'une composition selon l'une quelconque des revendications 1 à 14 dans la préparation d'un médicament visant à retarder la vitesse de croissance de cellules néoplasiques.

19. Utilisation d'une composition selon l'une quelconque des revendications 1 à 14 dans la préparation d'un médicament destiné à traiter des cellules néoplasiques animales ou humaines.

20. Utilisation d'une composition selon l'une quelconque des revendications 1 à 14 dans la préparation d'un médicament destiné à prévenir la récidive d'une pathologie néoplasique.

21. Utilisation d'une composition selon l'une quelconque des revendications 1 à 14 dans la préparation d'un médicament visant à prolonger le temps de survie d'un hôte abritant ladite cellule néoplasique.

22. Méthode *in vitro* de traitement d'une cellule néoplasique, la méthode comprenant l'étape consistant à administrer aux cellules une dose efficace d'une composition selon l'une quelconque des revendications 1 à 14.

23. Méthode selon la revendication 22, dans laquelle ladite cellule néoplasique est une cellule de moelle osseuse.

24. Utilisation d'une composition selon l'une quelconque des revendications 1 à 14 dans la préparation d'un médicament visant à détruire les cellules néoplasiques dans la moelle osseuse.

**25.** Utilisation d'une composition selon la revendication 24, dans laquelle les cellules de moelle osseuse surexpriment la protéine c-erbB-2.

FIG. 1

EP 0 635 030 B1

FIG. 2

FIG. 3

FIG. 4

1 ug/ml TAb 250
0.01 ug/ml TAb 250
1 ug/ml TAb 250-GELONIN
0.01 ug/ml TAb 250-GELONIN

FIG. 5

FIG. 6A

FIG. 6B

FIG. 6C

FIG. 6D

EP 0 635 030 B1

FIG. 7